# EUROPEAN PATENT SPECIFICATION

(11) **EP 0 490 852 B1**
(45) Date of publication and mention of the grant of the patent: **17.04.1996**
(21) Application number: 91850302.0
(22) Date of filing: 03.12.1991
(51) Int. Cl.: A61M 16/04

(54) **Tracheal tube**
Trachealtubus
Tube trachéal

(30) Priority: 10.12.1990 SE 9003934
(43) Date of publication of application: 17.06.1992
(73) Proprietor: MEDICAL PRODUCTS OCTAGON AB, S-751 45 Uppsala (SE)
(72) Inventor: Lindholm, Carl-Erik, S-756 46 Uppsala (SE)
(74) Representative: Sundström, Per Olof

(56) References cited:
- FR-A- 2 453 634
- US-A- 3 756 244
- US-A- 4 056 104
- "The Conformity of an Anatomically Shaped Endotracheal Tube to the Shape of the Airway", Acta Anaesthesiol Scand 1983:27:335-338.

## Description

The present invention relates to a tracheal tube of the kind set forth in the preamble of the following Claim 1, i.e. a tracheal tube which is comprised of a generally curved flexible tube made of plastic or some other material, having a patient-end which is inserted through the larynx and down into the trachea or windpipe, via the patient's mouth or nose. Mounted adjacent the patient-end of the tube is an inflatable collar or cuff which effects a seal between the tube and the trachea.

The standpoint of techniques is represented by the endotracheal tube of modified curvature in the direction of its longitudinal axis disclosed in the article "The Conformity of an Anatomically Shaped Endotracheal Tube to the Shape of the Airway", Acta Anaesthesiol Scand 1983:27:335-338. The preamble of claim 1 defines the features known from this article

One problem with the use of (endo)tracheal tubes is that the tube is liable to inflict injury to the larynx, more specifically on the medial sides of the arytenoid cartilages. These injuries are mainly pressure injuries. Scars are formed when these injuries heal and the resultant scar tissue damages the articular regions between the arytenoid cartilages and the cricoid cartilage. One consequence is that the airway is restricted so as to cause difficulties in breathing, these difficulties even being life-threatening at times. The nature of the patient's voice can also be affected by the injuries inflicted, since the positions of the vocal cords are controlled by the arytenoid cartilages.

With the intention of reducing injury to the patient, attempts have been made to reduce the diameter of the tube. This reduction in diameter, however, has not been able to solve the aforedescribed problem satisfactorily and, instead, has resulted in a troublesome increase in the resistance to air flow through the tube.

Injuries of the aforedescribed kind have been observed when using tubes having an inner diameter of 8 mm in the case of males and an inner diameter of 7 mm in the case of females. The wall thickness of the tubes normally varies between 1.2-1.5 mm.

It has earlier been proposed to change the cross-sectional shape of the tube, from the conventional circular shape to the general shape of an isosceles triangle with the apex of the triangle turned frontwards, probably with the intention of adapting the tube cross-section to the triangular opening defined by the vocal cords. This change in tube cross-section, however, did not produce the desired result and has therefore been abandoned.

The object of the present invention is to provide a novel tracheal tube which solves the aforesaid problem of pressure injury to the larynx, while maintaining a tube cross-section of large external area and therewith enabling a correspondingly large flow of air to pass through the tube.

This object is achieved with the tracheal tube defined in the following Claim 1.

The invention starts form a known tube having a general curved shape, wherein the tube leg or tube end which is intended to extend through the larynx may have a slight curvature which is inverted in relation to the main curvature of the tube. The inventive orientation and shape, particularly the pear-shaped or egg-shaped cross-section of the tube in that longitudinal section thereof which extends through the larynx, greatly reduces the aforesaid problem while still enabling the use of a large tube cross-section area which is favourable from the aspect of air flow.

The more pointed end of the generally pear-shaped tube cross-section shall thus be located on that side of the curved tube which is convex with respect to the main curvature of the tube, so that when the tube is inserted in position, the more pointed end of the tube cross-section will face rearwardly, i.e. towards the plate of the cricoid cartilage, and is received in the space between the arytenoid cartilages. The other, more obtuse end of the elongated egg-shaped or pear-shaped cross-section is turned towards the apex of the mutually converging vocal cords. The obtuse end of the cross-section is rounded so as to pass inwardly of and conform to the curvature of the forward inner wall of the cricoid cartilage downwardly of the arytenoid cartilages. The narrower, more pointed end of the cross-section is formed so as to pass in between the arytenoid cartilages without exerting local clamping pressure thereon. The more pointed end of the cross-section thus has approximately the same pointedness and width as the space located between the arytenoid cartilages, when the more pointed end of the cross-section rests generally on the inner wall of the cricoid cartilage.

The more narrow end of the tube cross-section is rounded so as to obtain an extended surface abutment with the underlying cricoid cartilage.

The outer large axis of the tube cross-section shall be at most equal to the inner diameter of the cricoid cartilage in the symmetry plane of the patient. The curvature of the preshaped tube is preferably such as to conform anatomically to the airway, as disclosed in the aforementioned article Acta Anaesthesiol Scand 1983:27:335-338, the teachings of which are incorporated in this document.

The flanks of the tube cross-section facing towards the arytenoid cartilages are generally parallel with the respective adjacent flanks of the arytenoid cartilages, when seen in a plane which is normal to the inserted tube in the region of the vocal cords. The tube flanks are preferably spaced apart at a distance such as not to exert any harmful pressure on the arytenoid cartilages. Thus, the rear, more pointed part of the tube cross-section inserted between the arytenoid cartilages will fill substantially the space defined in said plane by the mutually facing flanks of the arytenoid cartilages and the section of the innerwall of the cricoid cartilage which bridges the base regions of the flanks of the arytenoid cartilages.

The forward part of the cross-section may be rounded circularly, so as to minimize pressure strain on the vocal cords while, at the same time, maximizing the cross-sectional area of the tube so as to ensure the largest possible flow of air therethrough.

The invention will now be described with reference to an exemplifying embodiment thereof and also with reference to the accompanying drawing.
- Figure 1: is a schematic side view of the inventive tracheal tube.
- Figure 2: is a sectional view of the tube shown in Figure 1 taken through the line II-II in said Figure, and also illustrates the anatomical surroundings of the inserted tube at the vocal cords.
- Figure 3: illustrates an exemplifying embodiment of the tube cross-section at the part thereof which passes through the larynx.

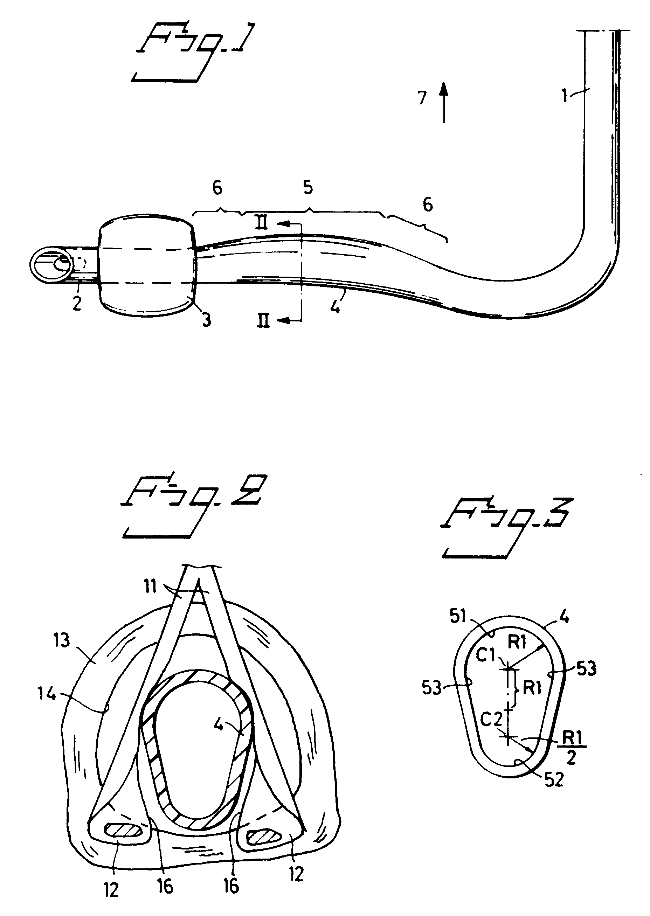

Figure 1 illustrates a piece of tubing which is curved in the plane of the drawing to a generally L-shape. One end or leg 1 of the tubing is intended to extend out through the patient's mouth or nose while the other end or leg 2 of the tubing is intended to extend down into the trachea or windpipe of the patient, through the patient's larynx, this second leg 2 of the tubing forming generally a right angle with the leg 1 and having a curvature which is inverse to the L-shaped curvature.

The extension of the tracheal tube formed by the tubing is designed to conform to the anatomical shape of the patient's airway, as described in more detail in Acta Anaesthisiol Scand 1983:27:335-338.

The leg 2 of the tube is surrounded by an inflatable collar or cuff 3, which can be expanded via channel devices (not shown) so as to form a seal between the outer surface of the leg 2 and the inner wall of the trachea. The tube-leg 2 has a particular cross-sectional shape and orientation at a longitudinal sectional 5 thereof which is intended to extend through the larynx region of the patient, as exemplified in Figures 2 and 3.

The tube preferably has a circular cross-section along the whole of its length, with the exception of the tube section 5 and transition zones 6 thereof which merge with the circular cross-section of the tube.

The arrow 7 shown in Figure 1 indicates a forward direction which refers to both the patient and a correctly positioned tube in the patient, this forward direction illustrating the direction to the front side of the patient.

Figure 2 shows the cross-sectional shape of the tube in the tube-section 5, in a plane which lies on the level of the vocal cords 11. The vocal cords 11 are attached to the arytenoid cartilages 12 at the rearward part of the larynx, and the cricoid cartilage 13, the inner wall of which is referenced 14, lies close beneath the vocal cords 11.

It will be seen that the mutually facing flanks of the arytenoid cartilages are substantially flat and diverge in the aforesaid forward direction 7, whereas the vocal cords converge in said direction 7. By giving the tube-section 5 of the tube an elongated cross-section with a more pointed part of the cross-section orientated in the curvature plane of the tube and in the patient's symmetry plane respectively, and by ensuring that the flanks of this more pointed part of the tube cross-section are generally parallel with the mutually facing flanks 16 of the arytenoid cartilages, it is possible to reduce the risk of pressure injury thereto, and by adapting the cross-sectional shape of the tube-section 5 in other respects to the limitations created by the vocal cords and the front wall of the cricoid cartilage, it is possible to optimize the cross-sectional area of the tube available for the passage of air therethrough.

Thus, an essential feature of the invention resides in giving the tube-section 5 an elongated shape, or pear-shape with the largest possible cross-sectional area, so as to enable a large volume of air to flow therethrough while, at the same time, minimizing pressure strains on primarily the arytenoid cartilages and only secondarily on the vocal cords.

It will be seen from Figure 3 that a favourable tube cross-section can be formed by a front circular arc of radius R1 and a rear circular arc of radius 0.5 x R1, wherein the curvature centres C1 and C2 of the two circular arcs are separated by a distance 1.5 x R1.

The flanks 53 of the cross-section are defined by tangents to the two circular arcs 51, 52.

In those embodiments in which the cross-section is pear-shaped, or similarly shaped, it is suitable to view the ovality of the front and the rear parts of the cross-section separately, each respective ovality being defined as the relationship between half the width and the full length of said part, and wherein the boundary between said front and said rear parts is defined by the position of the largest small-axis dimension.

In Figure 3, the ovality of the cross-sectional shape is 1:2 in the case of the rear part of the cross-section, although it will be understood that the ovality may generally lie in the range of 1:3 to 1:1.3. The front part of the cross-sectional shape is preferably delimited by a circular arc, although it will be understood that the front part of said cross-sectional shape may follow any other contour, for instance an elongated contour which can be accommodated favourably in the space defined by the mutually converging vocal cords and the front wall of the cricoid cartilage, as indicated in Figure 2.

The inventive tube may be formed from a length of tubing of circular cross-section and the cross-section of the tubing deformed to the illustrated shape in tube-section 5. In the simplest case, the circumference of the tube may be kept substantially constant, even in the tube-section 5.

It can be seen from Figure 1 that the rear contour of the tube is maintained unchanged also in the tube-section 5, and that the dimensions of the cross-section thus increase in the aforesaid forward direction 7.

In the case of males, the piece of tubing from which the tube is formed may have an inner diameter of 8 mm, wherein the outer diameter will be approximately 10.5 mm, i.e. the tubing has a wall thickness of about 1.3 mm. This tube cross-section will exert an acceptably low resistance to air flow, even when taking into account the deformed cross-section in the tube-section 5, this deformation of the tube cross-section reducing the risk of injury to the arytenoid cartilages and the vocal cords. In the case of males, the average distance between the articular surfaces of the cricoid cartilage that face towards the arytenoid cartilages is about 10.6 mm. Accordingly, it has been found that a piece of tubing having an inner diameter of 8 mm and a wall thickness of 1.3 mm is suitable as a manufacturing blank for manufacture of the inventive tube. Correspondingly, a piece of tubing having an inner diameter of 7 mm and a wall thickness of 1.3 mm can be used as a manufacturing blank for tubes intended for females.

In the case of babies and infants, for example premature babies, the tube blank may have an inner diameter of 2 mm and a wall thickness which is smaller than about 1 mm.

The length of the tube-section 5 is about four times the outer diameter of the round tube.

The transition zones 6 may have a length of about 15 mm, at least in the case of adult patients.

In the case of the Figure 1 embodiment, a side-hole is provided in the patient-end of the tube (in accordance with Murphy), although the provision of such a side-hole is not absolutely necessary.

The elongated shape of the cross-section 4 shall be rounded. By ovality is meant the width-length ratio of the tube cross-section or a corresponding ratio for a part of said cross-section.

A preferred tube material is PVC of the kind used at present by large manufacturers of tracheal tubes, or silicone, for use for the purpose concerned here.

## Claims

1. A tracheal tube comprising a generally curved flexible tube which includes a first end (2) which is intended for insertion into the patient's trachea, through the mouth or the nose of the patient and the patient's larynx, and a second end (1) which is intended to extend out through the patient's mouth or nose, and a curved tube-section joining said ends (1, 2), characterized in that the first tube end (2) in a portion (5) thereof which is intended to extend through the larynx region, has an elongated cross-section (4) with a major axis lying in a plane containing the longitudinal axes of the ends (1, 2) of said tube; in that said elongated cross-section (4) is substantially egg-shaped or pear-shaped to be more pointed at one of the major axis ends than at the other; and in that the more pointed end part of said cross-section is turned rearwardly towards the convex side of the main curvature of said tube.

2. A tracheal tube according to Claim 1, characterized in that the tube-portion (5) intended to extend through the larynx region of the patient has an ovality in the region of 1:3 to 1:1.3, calculated for the inner wall of the tube, preferably 1:2, at least at that more pointed part thereof which faces rearwardly so as to be located between the arytenoid cartilages.

3. A tracheal tube according to Claim 2, characterized in that the forwardly turned part of the elongated tube cross-section (4) is essentially semicircular in shape.

4. A tracheal tube according to Claim 2 or 3, characterized in that the tube has a wall thickness in the region of 0.8-1.5 mm, preferably 1.3 mm.

5. A tracheal tube according to any one of Claims 1 to 4, characterized in that the tube-portion (5) which presents the elongated cross-section and is intended to extend through the larynx has a length which is equal to about four times the outer diameter of the tube.

6. A tracheal tube according to any one of Claims 1 to 5, characterized in that the tube is generally L-shaped, and in that the medial part of the first end (2) has an inverse bend in relation to the main curvature between the ends of the L-shaped tube.

## Patentansprüche

1. Ein Trachealtubus enthaltend einen im allgemeinen gebogenen flexiblen Tubus, welcher ein erstes Ende (2) enthält, welches zur Einführung durch den Mund oder die Nase des Patienten und den Larynx des Patienten in die Trachea des Patienten vorgesehen ist, und ein zweites Ende (1), welches sich durch den Mund oder die Nase des Patienten herauserstrecken soll, und einen gebogenen Tubus-Abschnitt, welcher die Enden (1, 2) verbindet, dadurch gekennzeichnet, daß das erste Tubus-Ende (2) in einem Abschnitt (5) davon, welcher sich durch den Larynx-Bereich erstrecken soll, einen verlängerten Querschnitt (4) mit einer Hauptachse hat, welche in einer Ebene liegt, welche die Längsachsen der Enden (1, 2) des Tubus enthält; daß der verlängerte Querschnitt (4) im wesentlichen eiförmig oder birnenförmig ist, um an einem der Enden der Hauptachse spitziger zu sein als am anderen; und daß der spitzigere Endteil des Querschnitts nach hinten zur konvexen Seite der Hauptkrümmung des Tubus hin gedreht ist.

2. Ein Trachealtubus gemäß Anspruch 1, dadurch gekennzeichnet, daß der Tubus-Abschnitt (5), welcher sich durch den Larynx-Bereich des Patienten erstrecken soll, eine Ovalität im Bereich von 1:3 bis 1:1,3 hat, berechnet für die innere Wand des Tubus, vorzugsweise 1:2; zumindest an dessen spitzigerem Teil, welcher nach hinten gerichtet ist, um zwischen den arytenoiden Knorpeln angeordnet zu werden.

3. Ein Trachealtubus gemäß Anspruch 2, dadurch gekennzeichnet, daß der nach vorne gedrehte Teil des verlängerten Tubus-Querschnitts (4) eine im wesentlichen halbkreisförmige Gestalt hat.

4. Ein Trachealtubus gemäß Anspruch 2 oder 3, dadurch gekennzeichnet, daß der Tubus eine Wanddicke im Bereich von 0,8-1,5 mm, vorzugsweise 1,3 mm hat.

5. Ein Trachealtubus gemäß einem der Ansprüche 1 bis 4, dadurch gekennzeichnet, daß der Tubus-Abschnitt (5), welcher den verlängerten Querschnitt darstellt und sich durch den Larynx erstrecken soll, eine Länge hat, welche ungefähr viermal dem äußeren Durchmesser des Tubus entspricht.

6. Ein Trachealtubus gemäß einem der Ansprüche 1 bis 5, dadurch gekennzeichnet, daß der Tubus im allgemeinen L-förmig ist und daß der mittlere Teil des ersten Endes (2) eine entgegengesetzte Biegung im Verhältnis zur Hauptkrümmung zwischen den Enden des L-förmigen Tubus hat.

## Revendications

1. Tube trachéal, constitué par un tube flexible généralement courbé qui comporte une première extrémité (2) destinée à être introduite dans la trachée du patient, par la bouche ou par le nez du patient et par le larynx du patient, et une seconde extrémité (1) qui est destinée à sortir par la bouche ou par le nez du patient, ainsi qu'une section de tube courbé réunissant lesdites extrémités (1,2), caractérisé en ce que la première extrémité du tube (2) dans sa partie (5) qui est destinée à traverser la région du larynx, a une setion transversale allongée (4) dont le grand axe se situe dans un plan contenant l'axe longitudinal des extrémités (1,2) dudit tube, et en ce que ladite section transversale allongée (4) a sensiblement la forme d'un oeuf ou d'une poire pour être plus pointue à l'une des extrémités du grand axe qu'à l'autre; et en ce que la partie d'extrémité la plus pointue de ladite section transversale est orientée vers l'arrière en direction du côté convexe de la courbure principale dudit tube.

2. Tube trachéal selon la revendication 1, caractérisé en ce que la partie (5) du tube destiné à traverser la région du larynx du patient a dans la région une forme ovale de 1:3 à 1:1,3 calculée pour la paroi intérieure du tube, et de préférence de 1:2, au moins dans la partie la plus pointue qui est orientée vers l'arrière de façon à pouvoir se loger entre les cartilages aryténoïdiens.

3. Tube tracheal selon la revendication 2, caractérisé en ce que la partie tournée vers l'avant de la section transversale allongée du tube (4) a essentiellement une forme semi-circulaire.

4. Tube trachéal selon la revendication 2 ou 3, caractérisé en ce que le tube a une épaisseur de paroi située dans la région allant de 0,8 à 1,5 mm et de préférence égale à 1,3 mm.

5. Tube trachéal selon l'une quelconque des revendications 1 à 4, caractérisé en ce que la partie de tube (5) qui présente la section transversale allongée et qui est destinée à traverser le larynx a une longueur qui est égale à environ 4 fois le diamètre extérieur du tube.

6. Tube trachéal selon l'une quelconque des revendications 1 à 5, caractérisé en ce que le tube a généralement la forme d'un L et en ce que la partie médiale de la première extrémité (2) a une courbure inverse de la courbure principale entre les extrémités du tube en forme de L.
